# EUROPÄISCHE PATENTSCHRIFT

(11) **EP 4 749 640 B1**
(45) Veröffentlichungstag und Bekanntmachung des Hinweises auf die Patenterteilung: **18.11.2026**
(21) Anmeldenummer: 25216317.5
(22) Anmeldetag: 17.11.2025
(51) Int. Cl.: G16H 20/40, G06N 3/08, G16H 30/40, G16H 40/20, G16H 40/63, G16H 70/20

(54) **VERFAHREN UND VORRICHTUNG ZUR DOKUMENTATION EINER OPERATION AN EINEM PATIENTEN**
METHOD AND DEVICE FOR DOCUMENTING A SURGERY ON A PATIENT
PROCÉDÉ ET DISPOSITIF DE DOCUMENTATION D'UNE INTERVENTION CHIRURGICALE SUR UN PATIENT

(30) Priorität: 21.11.2024 DE 102024134270
(43) Veröffentlichungstag der Anmeldung: 27.05.2026
(73) Patentinhaber: FORBENCAP GmbH, 87527 Sonthofen (DE)
(72) Erfinder: Conle, Robert Ludwig, 87527 Sonthofen (DE)
(74) Vertreter: advotec.

(56) Entgegenhaltungen:
- US-A1- 20240 212 812
- CHANDANA K R ET AL: "Real Time Video Based Detection Of Retained Surgical Instruments During Intraoperative Procedures", 2024 CONTROL INSTRUMENTATION SYSTEM CONFERENCE (CISCON), IEEE, 2 August 2024 (2024-08-02), pages 1 - 6, XP034715677, DOI: 10.1109/CISCON62171.2024.10696273

## Beschreibung

Die Erfindung betrifft ein Verfahren und eine Vorrichtung zur automatisierten Dokumentation einer Operation eines Patienten.

### Stand der Technik

Die Dokumentation von Operationen ist ein entscheidender Bestandteil moderner medizinischer Prozesse, da sie sowohl der Nachvollziehbarkeit und Qualitätssicherung dient als auch rechtliche und medizinische Sicherheit gewährleistet. Diese Dokumentation wird bisher überwiegend manuell durchgeführt, was zeitaufwendig ist und wertvolle Ressourcen des OP-Teams beansprucht. Digitale Ansätze, wie die Nutzung von Tablets oder anderen Eingabegeräten, werden zwar zunehmend eingesetzt, erfordern jedoch weiterhin eine erhebliche menschliche Interaktion.

Mit der zunehmenden Komplexität von Operationen und dem wachsenden Druck zur Effizienzsteigerung steigt die Notwendigkeit einer präzisen und gleichzeitig zeitsparenden Dokumentation. Eine Reduzierung des Dokumentationsaufwands könnte es den Operateuren ermöglichen, sich verstärkt auf die eigentliche Durchführung der Operationen zu konzentrieren und gleichzeitig die Qualität der Dokumentation zu verbessern.

Weiterer Stand der Technik kann dem Dokument US 2024 0 212 812 A1 entnommen werden, das ein Verfahren und ein System zum Erzeugen eines medizinischen Berichts für medizinische Behandlungen offenbart.

Es ist eine Aufgabe der Erfindung, ein Verfahren und/oder eine Vorrichtung zur automatisierten Dokumentation von Operationen eines Patienten bereitzustellen, die den manuellen Aufwand minimiert und die Präzision der Dokumentation erhöht.

### Offenbarung der Erfindung

Die Aufgabe wird durch ein Verfahren gemäß Anspruch 1 sowie durch eine Vorrichtung gemäß Anspruch 9 gelöst.

Gemäß einem bevorzugten Aspekt wird ein Verfahren zur automatisierten Dokumentation einer Operation eines Patienten vorgeschlagen. Dieses Verfahren umfasst die Schritte: Erfassen von Bilddaten von Interaktionen zwischen einem Operateur und/oder einem Operationsutensil und einem Patienten unter Verwendung einer Bildaufnahmeeinheit, Verarbeiten der erfassten Bilddaten unter Verwendung eines Bildverarbeitungsalgorithmus zum Erkennen der Interaktionen in den Bilddaten sowie Generieren von Text-und/oder Sprachlabels zu den erkannten Interaktionen unter Verwendung eines maschinellen Labelgenerierung-Lernmodells. Ferner umfasst das Verfahren ein Erzeugen einer Warnmeldung unter Verwendung der generierten Text- und/oder Sprachlabels, wenn eine Gefahr besteht, dass ein Operationsutensil in einem Körper des Patienten verbleiben könnte.

Das Verfahren sieht vor, dass Bilddaten von Interaktionen zwischen einem Operateur und/oder einem Operationsutensil und einem Patienten erfasst werden. Diese Bilddaten dokumentieren vorzugsweise typische Handlungen während einer Operation, wie die Vorbereitung eines Operationsutensils, die Gewebepräparation, das Platzieren eines Implantats und/oder die Durchführung einer Naht. Die Bildaufnahmeeinheit kann dabei in Form einer tragbaren Kamera, beispielsweise integriert in einer OP-Brille, oder als stationär im Operationssaal angeordnetes Gerät ausgeführt sein. Alternativ könnte die Bildaufnahmeeinheit auch Tiefenkameras oder multispektrale Kameras umfassen, um zusätzliche Details der Interaktionen zu erfassen. Die Bildaufnahmeeinheit kann vorzugsweise auch Teil bzw. Bestandteil einer vorhandenen OP-Ausstattung sein. So kann beispielsweise eine bei einem mikroinvasiven Operationsgerät vorhandene Bildaufnahmeeinheit auch für den vorliegenden Anwendungsfall (mit) genutzt werden. Dabei werden vorzugsweise die von der Bildaufnahmeeinheit erzeugten bzw. erzeugbaren Bilddaten auf vorliegend beschriebene Art und Weise verarbeitet, um die automatische OP-Dokumentation zu erstellen.

Weiterhin erfolgt die Verarbeitung der erfassten Bilddaten, wobei ein Bildverarbeitungsalgorithmus eingesetzt wird, um die in den Bilddaten enthaltenen Interaktionen zu erkennen. Ein solcher Algorithmus kann beispielsweise auf Verfahren wie Segmentierung, Objekterkennung oder Bewegungsanalyse basieren. Zusätzlich oder alternativ könnten Ansätze zur Aktivitätserkennung, Anomalieerkennung oder Objektverfolgung integriert werden, um komplexe Operationsschritte zu identifizieren oder kritische Situationen, wie das Risiko, dass ein Operationsutensil im Körper des Patienten verbleibt, zu detektieren.

In einem weiteren Schritt des Verfahrens werden aus den erkannten Interaktionen Text-und/oder Sprachlabels generiert. Dies erfolgt durch ein maschinelles Lernmodell, vorzugsweise ein Transformer-Modell, das zuvor anhand annotierter Operationsprotokolle und Bilddaten trainiert wurde. Die Labels beschreiben vorzugsweise die durchgeführten Operationsschritte präzise und, insbesondere in zeitlicher Abfolge, strukturiert und können als Text und/oder in Form von Audio ausgegeben werden. Alternativ oder ergänzend könnten auch andere Lernmodelle, wie Recurrent Neural Networks (RNNs) oder Convolutional Neural Networks (CNNs), eingesetzt werden, um spezifische Aufgaben wie die Analyse zeitlicher Bildsequenzen zu bewältigen.

Zusätzlich könnte das Verfahren eine Warnmeldung generieren, wenn eine erkannte Interaktion von einem standardisierten Operationsprotokoll abweicht, und so die Sicherheit während der Operation erhöhen.

Das vorgeschlagene Verfahren bietet zahlreiche technische Vorteile. Es reduziert den manuellen Aufwand für die Dokumentation von Operationen, was den Operateuren und dem OP-Team mehr Zeit für die direkte Durchführung und Überwachung der Operationen gibt. Durch den Einsatz maschineller Lernmodelle wird eine hohe Präzision und Konsistenz bei der Dokumentation gewährleistet. Die automatisch generierten Daten können vorzugsweise revisionssicher gespeichert und bei Bedarf überprüft werden, um den Operationsverlauf nachvollziehbar zu machen und potenzielle Risiken zu minimieren. Die Flexibilität des Verfahrens ermöglicht eine Anpassung an verschiedene Operationsumgebungen und/oder -szenarien, während die Skalierbarkeit eine Implementierung in Einrichtungen unterschiedlichster Größe, von kleinen ambulanten OP-Zentren bis hin zu großen Krankenhäusern, unterstützt. Darüber hinaus gewährleistet die Möglichkeit zur Anonymisierung der Daten die Einhaltung datenschutzrechtlicher Anforderungen, insbesondere bei der Verarbeitung sensibler Patientendaten.

Es versteht sich, dass die erfindungsgemäßen Schritte sowie weitere optionale Schritte nicht notwendigerweise in der aufgezeigten Reihenfolge ausgeführt werden müssen, sondern auch in einer anderen Reihenfolge oder parallel ausgeführt werden können. Ferner können weitere Zwischenschritte vorgesehen sein. Die einzelnen Schritte können zudem einen oder mehrere Unterschritte umfassen, ohne dass hierdurch der Umfang des erfindungsgemäßen Verfahrens verlassen wird.

Das maschinelle Lernmodell generiert vorzugsweise Labels automatisch aus Bilddaten, indem es während des Trainings gelernt hat, visuelle Muster, Bewegungen und Kontextinformationen mit spezifischen Bedeutungen zu verknüpfen. Dieser Prozess erfolgt vorzugsweise in mehreren Schritten, die ineinandergreifen. Zunächst könnten die erfassten Bilddaten vorverarbeitet werden, um sie für das Modell konsistent und leichter verarbeitbar zu machen. Dazu gehört vorzugsweise die Anpassung der Bildgröße, die Normalisierung von Farbräumen und die Entfernung von Bildrauschen. Für Videodaten könnten vorzugsweise Sequenzen von Frames extrahiert werden, um zeitliche Abläufe, wie beispielsweise Bewegungen von Operationsutensilien oder spezifische Handgriffe, zu analysieren. Das maschinelle Lernmodell weist vorzugsweise ein Sprachmodell, insbesondere ein großes Sprachmodell, auf.

Das maschinelle Lernmodell kann ein GPT (Generative Pre-trained Transformer) aufweisen, das auf der Transformer-Architektur basiert und besonders gut für die Verarbeitung von Texten und die Generierung von natürlich klingender Sprache geeignet ist. Das maschinelle Lernmodell kann auch ein BERT (Bidirectional Encoder Representations from Transformers) aufweisen, das ebenfalls auf der Transformer-Architektur basiert und sich durch seine Fähigkeit auszeichnet, kontextuelle Zusammenhänge in Texten bidirektional zu analysieren. Alternativ kann das maschinelle Lernmodell ein T5 (Text-to-Text Transfer Transformer) aufweisen, das ebenfalls eine Transformer-Architektur verwendet und sich darauf spezialisiert hat, jede Textverarbeitungsaufgabe als Text-zu-Text-Problem zu formulieren.

Weiterhin kann das maschinelle Lernmodell ein XLNet aufweisen, das auf einer Transformer-Architektur mit autoregressiven und autoencoderartigen Mechanismen basiert und kontextuelle Abhängigkeiten besser modellieren kann. Ein weiteres mögliches Modell kann RoBERTa (Robustly Optimized BERT Approach) aufweisen, eine optimierte Version von BERT, die durch umfangreicheres Training auf größeren Datensätzen eine höhere Leistungsfähigkeit erreicht. Das Modell könnte auch ein ALBERT (A Lite BERT) sein, eine leichte und optimierte Variante von BERT, die weniger Speicherplatz benötigt und schneller trainierbar ist.

Die Anwendung dieser Modelle ermöglicht eine präzise und strukturierte Dokumentation der erkannten Interaktionen, was die Qualität und Konsistenz der Operationsprotokolle erheblich verbessert.

Darüber hinaus kann das maschinelle Lernmodell ein OpenAI Codex aufweisen, ein Transformer-basiertes Modell, das speziell für die Verarbeitung und Generierung von Code trainiert wurde. Für multimodale Anwendungen kann ein CLIP (Contrastive Language-Image Pre-training) Modell eingesetzt werden, das auf einer Transformer-Architektur basiert und Text- und Bildinformationen kombiniert, um visuelle und sprachliche Eingaben miteinander in Beziehung zu setzen. Schließlich könnte ein Transformer-XL verwendet werden, eine erweiterte Transformer-Architektur, die längere kontextuelle Abhängigkeiten modellieren kann.

Das Modell extrahiert vorzugsweise visuelle Merkmale aus den Daten. In den ersten Schichten eines neuronalen Netzwerks, beispielsweise in Convolutional Neural Networks (CNNs) oder Transformer-Modellen, könnten grundlegende Muster wie Kanten, Farben oder Texturen erkannt werden. Fortgeschrittene Schichten abstrahieren vorzugsweise diese Merkmale weiter und identifizieren komplexere Strukturen wie Objekte oder spezifische Handlungen, etwa das Halten eines Operationsutensils oder das Platzieren eines Implantats.

Mithilfe von Objekterkennungs- und Szenenanalyse-Algorithmen wie YOLO oder Mask R-CNN könnten vorzugsweise einzelne Objekte in den Bildern oder Videos erkannt und segmentiert werden. Diese Analyse ermöglicht es vorzugsweise, spezifische Operationstätigkeiten wie das Anreichen eines Skalpells oder das Einsetzen eines Katheters in ihrem visuellen Kontext zu erkennen. Für dynamische Szenen, in denen Bewegungen entscheidend sind, könnte das Modell Bewegungen über mehrere Frames hinweg analysieren. Dabei könnten vorzugsweise optische Flussalgorithmen oder 3D-CNNs genutzt werden, um Aktivitäten wie das Nähen einer Wunde oder die Entfernung eines Fremdkörpers zu identifizieren.

Um die Informationen sinnvoll zu verknüpfen, interpretiert das Modell vorzugsweise die erkannten Muster und Bewegungen im Kontext der Operation. Transformer-Modelle oder Recurrent Neural Networks (RNNs) könnten vorzugsweise eingesetzt werden, da sie zeitliche und räumliche Zusammenhänge berücksichtigen. So könnte das Modell erkennen, ob der Operateur gerade ein Operationsutensil vorbereitet oder eine spezifische Handlung am Patienten ausführt. Diese kontextuelle Interpretation ist vorzugsweise entscheidend, um präzise Labels zu generieren.

Im letzten Schritt könnten die Ergebnisse der Objekterkennung, Bewegungsanalyse und Kontextinterpretation vorzugsweise mit zuvor erlernten Operationsprotokollen abgeglichen werden. Das Modell könnte basierend auf dieser Analyse Labels erstellen, die die erkannte Handlung oder Beobachtung beschreiben, wie etwa "Operationsbesteck sterilisiert", "Implantat platziert" oder "Schnittwunde geschlossen".

Das Modell kann diese Labels vorzugsweise automatisch generieren, weil es auf annotierten Datensätzen trainiert wurde, die Bilder und Videos mit präzisen Beschreibungen enthalten. Während des Trainingsprozesses könnte es lernen, wie spezifische visuelle Muster, wie etwa die Bewegung eines Skalpellträgers oder die Position einer Klemme, mit bestimmten Operationstätigkeiten korrelieren. Darüber hinaus könnten Transformer-Modelle vorzugsweise Informationen aus mehreren Quellen kombinieren, etwa aus Bilddaten, Bewegungsmustern und Umgebungsinformationen, um eine kontextuelle Interpretation der Szene zu ermöglichen.

Ein praktisches Beispiel für die automatische Labelgenerierung könnte vorzugsweise das Platzieren eines Implantats sein. Eine Kamera könnte aufzeichnen, wie der Operateur das Implantat an die vorgesehene Position bringt. Das Modell könnte dabei den Operateur, das Implantat und die Bewegungssequenz erkennen und vorzugsweise das Label "Implantat erfolgreich platziert" generieren. In einem anderen Szenario könnte das Modell die Bewegungen des Operateurs und eines Assistenten während einer Naht analysieren und vorzugsweise das Label "Wundverschluss durchgeführt" erzeugen. Auch bei der Verwendung von bildgesteuerten Techniken, wie der Navigation eines Katheters, könnte das Modell automatisch das Utensil, die Position des Patienten und die Interaktion erkennen, um vorzugsweise das Label "Katheter eingeführt" zu erstellen.

In einem weiteren bevorzugten Aspekt wird eine Vorrichtung zur automatisierten Dokumentation von Operationen eines Patienten vorgeschlagen, wobei die Vorrichtung eine Auswerte- und Recheneinrichtung aufweist, die dazu ausgebildet ist, die folgenden Schritte auszuführen: Erfassen von Bilddaten von Interaktionen zwischen einem Operateur und/oder einem Operationsutensil und einem Patienten unter Verwendung einer Bildaufnahmeeinheit; Verarbeiten der erfassten Bilddaten unter Verwendung eines Bildverarbeitungsalgorithmus zum Erkennen der Interaktionen in den Bilddaten; Generieren von Text- und/oder Sprachlabels zu den erkannten Interaktionen unter Verwendung eines maschinellen Lernmodells, insbesondere eines Transformer-Modells; und Erzeugen einer Warnmeldung unter Verwendung der generierten Text- und/oder Sprachlabels, wenn eine Gefahr besteht, dass ein Operationsutensil in einem Körper des Patienten verbleiben könnte.

Die für das Verfahren gemachten Ausführungen gelten für die Vorrichtung entsprechend. Dabei versteht es sich, dass sprachliche Abwandlungen von verfahrensmäßig formulierten Merkmalen nach sprachüblicher Praxis für die Vorrichtung umformulierbar sind, ohne dass derartige Formulierungen explizit hier aufgeführt werden müssen.

In einem weiteren Aspekt wird vorgeschlagen, dass das Verfahren eine Bildaufnahmeeinheit umfasst bzw. nutzt, die eine Kamera und/oder ein tragbares Gerät zur Bilderfassung, wie eine OP-Brille oder ein anderes tragbares Kamerasystem, aufweist oder auf Bilddaten einer bereits im Einsatz befindlichen Bildaufnahmeeinheit zugreift.

Die Bildaufnahmeeinheit ermöglicht vorzugsweise die Erfassung visueller Daten der Interaktionen zwischen einem Operateur und/oder einem Operationsutensil und einem Patienten während einer Operation. Dabei kann die Kamera stationär im Operationssaal, beispielsweise an einer OP-Leuchte oder am OP-Tisch, installiert sein oder mobil verwendet werden. Tragbare Geräte wie OP-Brillen erhöhen die Flexibilität des Operateurs und können kontextrelevante Daten wie die Blickrichtung erfassen. Alternativ oder ergänzend könnten tragbare Geräte wie Körperkameras verwendet werden, die direkt am Operateur oder am Operationsutensil angebracht sind. Diese Merkmale erleichtern vorzugsweise die Integration in die Arbeitsabläufe im Operationssaal, ohne die Durchführung der Operation zu behindern. Die Verwendung tragbarer Bildaufnahmeeinheiten ermöglicht die Erfassung von Bilddaten in unmittelbarer Nähe der Operationshandlung, was die Präzision der Dokumentation erhöht. Tragbare Geräte minimieren den Platzbedarf und maximieren die Mobilität, während stationäre Einheiten eine kontinuierliche und umfassende Erfassung des OP-Geschehens sicherstellen.

In einem weiteren Aspekt wird vorgeschlagen, dass das Verfahren eine Bildaufnahmeeinheit umfasst, die stationär oder beweglich in einem Operationsraum installiert ist und/oder an einem Körper des Operateurs oder an einem Operationsutensil angeordnet ist.

Die Anordnung der Bildaufnahmeeinheit bestimmt vorzugsweise die Perspektive und Reichweite der erfassten Daten. Stationäre Einheiten, wie Kameras, die an der Decke oder einer OP-Leuchte montiert sind, ermöglichen eine umfassende Überwachung des Operationsbereichs. Bewegliche Bildaufnahmeeinheiten wie tragbare Kameras oder Geräte, die an OP-Besteck angebracht sind, können vorzugsweise flexibel positioniert werden, um spezifische Details der Interaktionen zu erfassen. Alternativ könnten Sensoren direkt an den Operationsutensilien oder am Körper des Operateurs angebracht werden, um eine personalisierte Erfassung zu gewährleisten. Stationäre Einheiten bieten eine dauerhafte, raumübergreifende Erfassung, während tragbare Geräte detaillierte Daten aus der Nähe dokumentieren. Körpergebundene Kameras liefern individuelle Perspektiven und reduzieren den Einfluss von Umgebungselementen. Die Vielseitigkeit der Anordnungsmöglichkeiten erhöht vorzugsweise die Funktionalität des Systems in unterschiedlichen Operationsumgebungen. Bewegliche Bilderfassungseinheiten verbessern die Einsatzfähigkeit bei komplexen oder wechselnden Operationsszenarien.

In einem weiteren Aspekt wird vorgeschlagen, dass der Bildverarbeitungsalgorithmus eine Segmentierung der Bilddaten und/oder eine Objekterkennung und/oder eine Bewegungsanalyse und/oder eine Aktivitätserkennung und/oder eine Anomalieerkennung und/oder eine Objektverfolgung und/oder eine Klassifikation von Handlungen und/oder eine multimodale Bildverarbeitung und/oder eine Feature-Extraktion aufweist.

Der Bildverarbeitungsalgorithmus analysiert vorzugsweise die erfassten Bilddaten und extrahiert spezifische Informationen. Segmentierung unterteilt das Bild vorzugsweise in relevante Bereiche, während Objekterkennung bestimmte Operationsutensilien, wie Skalpelle oder Klemmen, identifiziert. Bewegungs- und Aktivitätserkennung dienen vorzugsweise der Analyse dynamischer Operationstätigkeiten, wie dem Schneiden, Nähen oder Platzieren eines Implantats. Anomalieerkennung könnte verwendet werden, um kritische Situationen wie das Risiko des Verbleibs eines Utensils im Patienten zu identifizieren. Multimodale Bildverarbeitung kombiniert vorzugsweise verschiedene Datentypen, wie RGB- und Tiefendaten, um eine umfassendere Analyse der Operation zu ermöglichen. Diese Algorithmen gewährleisten eine präzise Erkennung von Operationstätigkeiten und erhöhen die Verlässlichkeit der Dokumentation. Die Integration mehrerer Algorithmen bietet vorzugsweise hohe Flexibilität und Anpassungsfähigkeit an unterschiedliche Operationsszenarien.

In einem weiteren Aspekt wird vorgeschlagen, dass der Bildverarbeitungsalgorithmus ein maschinelles Lernmodell zur Bildverarbeitung, insbesondere ein neuronales Faltungsnetzwerk (CNN), aufweist.

Neuronale Faltungsnetzwerke (CNNs) eignen sich besonders für die Bildverarbeitung und können Muster und Objekte in Bilddaten effizient erkennen. Alternativ oder ergänzend könnten Transformer-Modelle oder hybride Ansätze genutzt werden, um komplexe zeitliche und räumliche Abhängigkeiten zu analysieren. Diese Modelle erhöhen die Genauigkeit und Geschwindigkeit der Bildverarbeitung und fördern die Automatisierung der Dokumentation.

Allgemein kann der Bildverarbeitungsalgorithmus ein maschinelles Lernmodell und/oder ein statistisches Modell und/oder ein analytisches Modell, insbesondere ein hybrides Modell, umfassend mehrere der vorgenannten Modelle in Kombination, aufweisen.

In einem weiteren Aspekt wird vorgeschlagen, dass das maschinelle Labelgenerierung-Lernmodell unter Verwendung von standardisierten Operationsprotokollen, der Operationshistorie sowie damit verbundenen Bilddaten trainiert oder zumindest feinabgestimmt wird.

Das Training des Modells auf spezifische und/oder vorbestimmte Operationsprotokolle ermöglicht vorzugsweise eine anwendungsspezifische Anpassung. Alternativ könnten öffentlich verfügbare oder synthetisch generierte Daten verwendet werden, um das Modell zu initialisieren. Das Training auf spezifische Daten erhöht vorzugsweise die Genauigkeit und Kontextsensitivität. Eine Feinabstimmung erlaubt eine personalisierte und kontextabhängige Dokumentation der Operation.

In einem weiteren Aspekt wird vorgeschlagen, dass eine Ausgabe der generierten Text-und/oder Sprachlabels als, insbesondere editierbare, Audio- und/oder Textdatei erfolgt.

Die Ausgabe in verschiedenen Formaten ermöglicht vorzugsweise eine einfache Integration in bestehende Systeme, wie elektronische Operationsprotokolle oder Krankenhausinformationssysteme (KIS). Alternativ können visuelle Darstellungen der Labels in Dashboards erfolgen, um dem OP-Team eine Übersicht der dokumentierten Schritte in Echtzeit zu bieten. Eine solche Flexibilität bei der Ausgabe fördert vorzugsweise die Nutzbarkeit und Anpassungsfähigkeit des Systems. Die editierbare Ausgabe erleichtert vorzugsweise Korrekturen und/oder Anpassungen an individuellen Anforderungen.

In einem weiteren Aspekt wird vorgeschlagen, dass fehlerhafte Labels in einer Revision der Operationsprotokolle identifiziert und nachfolgend dazu verwendet werden, das maschinelle Labelgenerierung-Lernmodell und/oder den Bildverarbeitungsalgorithmus nachzutrainieren.

Die kontinuierliche Verbesserung des Modells durch fehlerhafte Labels fördert vorzugsweise die Lernfähigkeit. Alternativ könnten externe Feedbackquellen, wie Anmerkungen von Operateuren oder Protokollprüfern, einbezogen werden. Nachtraining erhöht die langfristige Genauigkeit und Robustheit. Das System bleibt adaptiv und passt sich veränderten Bedingungen oder neuen Protokollen an.

In einem weiteren Aspekt wird vorgeschlagen, dass ein Computerprogrammprodukt umfassend Befehle enthält, die bei der Ausführung des Programms durch einen Computer diesen veranlassen, die Schritte des vorliegenden Verfahrens nach einer beliebigen Ausführungsform auszuführen.

Das Computerprogrammprodukt bildet die Grundlage für die Implementierung der Verfahren. Die Verfügbarkeit als Softwareprodukt erleichtert die Verbreitung und Integration in bestehende OP-Systeme. Ein einfacher Einsatz auf vorhandener Hardware wird ermöglicht.

Ein Computerprogramm, das die Schritte eines Verfahrens zur automatisierten Dokumentation von Operationen umsetzt, kann vorzugsweise modular aufgebaut sein und aus mehreren Komponenten bestehen, die spezifische Aufgaben übernehmen. Es könnte beispielsweise aus den folgenden Modulen bestehen:
Ein Eingabemodul dient vorzugsweise der Integration der Bildaufnahmeeinheit, die entweder stationär, tragbar oder beweglich ist. Dieses Eingabemodul steuert vorzugsweise die Erfassung der Bilddaten, synchronisiert die Aufnahmen bei Bedarf mit anderen Datensätzen (z. B. Zeitstempeln oder Patienteninformationen) und/oder bereitet die Bilddaten für die Verarbeitung vor. Zusätzlich kann dieses Eingabemodul Filter anwenden, um die Bildqualität zu optimieren, und datenschutzfreundliche Techniken wie Anonymisierung oder Unschärfe sensibler Daten integrieren.

Ein Bildverarbeitungsmodul analysiert vorzugsweise die erfassten Bilddaten mithilfe eines oder mehrerer Bildverarbeitungsalgorithmen. Hierbei könnten neuronale Netzwerke wie Convolutional Neural Networks (CNNs) oder Vision Transformers (ViTs) zur Objekterkennung, Segmentierung, Bewegungsanalyse oder Aktivitätserkennung eingesetzt werden. Dieses Bildverarbeitungsmodul ist vorzugsweise darauf ausgelegt, relevante Informationen wie Handlungen des Operateurs, den Einsatz von Operationsutensilien oder den Fortschritt der Operation zu extrahieren.

Ein Labelgenerierungsmodul verarbeitet vorzugsweise die Ergebnisse des Bildverarbeitungsmoduls und erstellt Text- und/oder Sprachlabels. Dieses Labelgenerierungsmodul nutzt vorzugsweise ein maschinelles Lernmodell, wie ein Transformer-Modell, das auf annotierten Operationsprotokollen und Bilddaten trainiert wurde. Die Labels werden vorzugsweise in strukturierter Form erstellt und können nach Bedarf als Text- oder Audioausgabe formatiert werden. Weiterhin kann das Labelgenerierungsmodul eine Feedback-Komponente beinhalten, die fehlerhafte Labels identifiziert und diese zur Optimierung des maschinellen Lernmodells nutzt.

Ein Ausgabemodul sorgt vorzugsweise für die Speicherung und Ausgabe der generierten Labels. Es könnte die Labels in verschiedenen Formaten bereitstellen, wie z. B. als editierbare Textdateien, Audioformate oder in Form einer visuellen Benutzeroberfläche, etwa einem Dashboard, das Echtzeit-Updates anzeigt. Dieses Ausgabemodul kann auch eine Schnittstelle zur Integration mit externen Systemen, wie elektronischen Operationsprotokollen oder Krankenhausinformationssystemen (KIS), bereitstellen.

Ein Trainings- und Anpassungsmodul ermöglicht vorzugsweise die Weiterentwicklung und Feinabstimmung der genutzten Modelle. Es könnte ein Nachtraining auf Basis neuer Daten oder fehlerhafter Labels durchführen und so die Präzision und Robustheit des Systems kontinuierlich verbessern. Dabei könnte es sowohl online (während der Nutzung) als auch offline (auf vorbereiteten Datensätzen) arbeiten, um neue Operationstechniken oder Protokollanforderungen zu integrieren.

Ein Sicherheitsmodul stellt vorzugsweise sicher, dass die Datenschutzanforderungen eingehalten werden. Es könnte Verschlüsselungstechniken zur Sicherung der gespeicherten Daten einsetzen und den Zugriff auf die Daten auf autorisierte Nutzer beschränken. Dieses Modul kann zudem Mechanismen zur Anonymisierung von sensiblen Informationen wie Gesichtern oder identifizierenden Merkmalen des Patienten integrieren, um datenschutzrechtliche Vorgaben zu erfüllen.

Das Verfahren zur automatisierten Dokumentation kann ferner dazu ausgebildet sein, unter Verwendung der generierten Text- und/oder Sprachlabels eine Warnmeldung zu erzeugen, wenn eine Interaktion von einem Operationsprotokoll abweicht. Diese Funktion dient der Erhöhung der Sicherheit während einer Operation und der Minimierung menschlicher Fehler.

Um Abweichungen zu erkennen, vergleicht das Verfahren die generierten Labels mit einem vorab definierten Operationsprotokoll. Das Operationsprotokoll enthält beispielsweise eine standardisierte, schrittweise Beschreibung der erforderlichen Tätigkeiten für den jeweiligen Eingriff, einschließlich der Reihenfolge und der erwarteten Zeiträume für bestimmte Schritte. Das System nutzt diese Informationen, um zu prüfen, ob die während der Operation dokumentierten Handlungen mit dem Protokoll übereinstimmen. Dabei können Abweichungen in zeitlicher Abfolge, inhaltlicher Ausführung oder Vollständigkeit erkannt werden. Wird beispielsweise ein Schritt übersprungen oder in einer nicht vorgesehenen Reihenfolge durchgeführt, generiert das System eine Warnmeldung, die auf die Abweichung hinweist.

Darüber hinaus überwacht das System den Umgang mit Operationsutensilien. Hierzu analysiert das maschinelle Lernmodell sowohl die visuell erfassten Bilddaten als auch die generierten Labels, um sicherzustellen, dass jedes verwendete Operationsutensil korrekt identifiziert und protokolliert wird. Am Ende der Operation kann das System automatisch prüfen, ob alle verwendeten Utensilien wie erwartet zurückgegeben wurden. Falls ein registriertes Operationsutensil fehlt, wird eine Warnmeldung ausgegeben, die das Operationsteam darauf hinweist, dass möglicherweise ein Utensil im Körper des Patienten verbleiben könnte. Dies kann beispielsweise durch einen Vergleich der initial erfassten Liste der genutzten Instrumente mit den am Ende registrierten zurückgelegten Instrumenten erfolgen.

Die Warnmeldungen werden vorzugsweise in Echtzeit ausgegeben und können visuell über eine Anzeigeeinheit oder auditiv als Sprachmeldung bereitgestellt werden. Visuelle Warnungen könnten beispielsweise auf einem Monitor im Operationssaal angezeigt werden, während akustische Warnungen über Lautsprecher oder tragbare Geräte an das Operationsteam gerichtet werden. Die Warnungen sind vorzugsweise präzise und geben konkrete Hinweise auf die Art der Abweichung oder das potenzielle Risiko, beispielsweise: "Schritt 4 des Protokolls wurde übersprungen" oder "Instrument X nicht zurückgegeben - Überprüfung erforderlich."

Für die Risikoerkennung nutzt das System vorzugsweise multimodale Datenfusion, bei der visuelle, textuelle und zeitliche Informationen kombiniert werden. Beispielsweise könnte das System durch eine kontinuierliche Analyse der Interaktionen und Bewegungen erkennen, ob ein Operationsutensil in einer ungewöhnlichen Position oder über einen unerwartet langen Zeitraum unbewegt bleibt. Solche Anomalien können ein Indikator dafür sein, dass ein Instrument versehentlich im Körper des Patienten verblieben ist. Wenn mit anderen Worten eine Anzahl von angereichten Utensilien nicht mit einer Anzahl von zurückgegebenen Utensilien übereinstimmt, wird vorliegend vorzugsweise ein Warnsignal erzeugt.

Die Flexibilität des Systems ermöglicht es, die Warnmeldungen vorzugsweise an spezifische Anforderungen oder Szenarien anzupassen. In Notfällen kann das System beispielsweise die Priorität der Warnmeldungen anpassen und besonders kritische Situationen hervorheben. Darüber hinaus kann das System durch maschinelles Lernen kontinuierlich verbessert werden, indem es aus vergangenen Warnmeldungen und deren Auflösungen lernt, um die Präzision und Relevanz der Warnmeldungen weiter zu steigern.

Die Integration dieser Funktion in das Verfahren trägt wesentlich zur Patientensicherheit bei, indem sie menschliche Fehler reduziert und das Operationsteam in kritischen Momenten unterstützt. Die Möglichkeit, potenziell lebensgefährliche Situationen frühzeitig zu erkennen, minimiert das Risiko von Komplikationen und trägt zu einer höheren Qualität und Zuverlässigkeit bei der Durchführung von Operationen bei.

Das Verfahren ist beispielsweise dazu ausgebildet, spezifische Warnmeldungen zu generieren, die kritische Situationen wie das Vergessen eines Tupfers im Körper des Patienten erkennen. Beispielsweise könnte das System eine Warnmeldung ausgeben, wenn festgestellt wird, dass die chirurgische Naht bereits begonnen hat, während noch nicht alle in den Körper eingebrachten Operationsutensilien, wie Tupfer, wieder entfernt wurden. Eine solche Warnmeldung könnte etwa lauten: "Achtung: Tupfer nicht entfernt - Überprüfung erforderlich, bevor die Naht abgeschlossen wird." Durch diese Funktion können potenziell schwerwiegende Folgeerkrankungen, wie innere Infektionen und Entzündungen, frühzeitig vermieden werden.

Das Verfahren zählt beispielsweise dazu kontinuierlich die Anzahl der in den Körper eingeführten Operationsutensilien, wie Tupfer, Schwämme und/oder Instrumente. Diese Anzahl wird während der gesamten Operation überwacht und beispielsweise mit der Anzahl der entnommenen Utensilien abgeglichen. Sollte eine Diskrepanz festgestellt werden, generiert das System eine Warnung und fordert das Operationsteam auf, eine Kontrolle durchzuführen. Dies erfolgt in Echtzeit, sodass entsprechende Maßnahmen ergriffen werden können, bevor die Operation fortgesetzt oder abgeschlossen wird.

Die Funktion basiert auf einer Kombination aus visueller Objekterkennung und Textlabels, die die Interaktionen des Operationspersonals mit den Utensilien dokumentieren. Beispielsweise könnte das maschinelle Lernmodell erkennen, wann ein Tupfer in den Körper eingeführt wird, und diese Handlung automatisch protokollieren. Ebenso wird erfasst, wenn der Tupfer den Körper wieder verlässt. Sollte die Anzahl der entnommenen Tupfer nicht mit der Anzahl der eingeführten übereinstimmen, wird dies umgehend detektiert.

Diese automatisierte Überwachung minimiert beispielsweise das Risiko menschlicher Fehler und erhöht die Sicherheit des Patienten erheblich. Zudem unterstützt das System das Operationsteam, indem es Routineaufgaben wie das Zählen der Utensilien übernimmt und präzise Warnmeldungen generiert. Dadurch werden Fehler reduziert, die Arbeitsbelastung des Teams verringert und eine höhere Präzision sowie Effizienz während der Operation gewährleistet.

In einem weiteren Aspekt wird vorgeschlagen, dass ein computerlesbarer Datenträger das vorliegende Computerprogrammprodukt speichert.

Der Datenträger dient vorzugsweise der langfristigen Speicherung und Verteilung des Programms. Die Speicherung auf Datenträgern sichert vorzugsweise die Portabilität und ermöglicht flexible Verteilung und Sicherung. Ein typischer Datenträger könnte ein physisches Medium wie eine CD, DVD oder Blu-ray-Disc sein, auf der das Programm dauerhaft gespeichert wird. Alternativ könnte ein Flash-basiertes Speichermedium wie ein USB-Stick oder eine SSD genutzt werden, das eine größere Speicherkapazität und einfachere Handhabung bietet.

Ein weiterer Ansatz wäre ein Cloud-basierter Datenträger, bei dem die Programmdaten auf einem entfernten Server gespeichert sind und über das Internet aufgerufen werden können. Solche Lösungen sind besonders geeignet für Szenarien, in denen Updates und gemeinsamer Zugriff auf das Programm erforderlich sind. In allen Fällen könnte der Datenträger zusätzliche Sicherheitsmechanismen wie Passwortschutz oder Verschlüsselung bieten, um unbefugten Zugriff zu verhindern.

Der Datenträger kann zudem so gestaltet sein, dass er direkt mit bestehenden Systemen, wie Krankenhausinformationssystemen, kompatibel ist und die nahtlose Integration ermöglicht. Dies fördert die einfache Verteilung und Skalierbarkeit des Programms in verschiedenen Operationsumgebungen.

Das Training des maschinellen Lernmodells für das vorliegende Verfahren kann vorzugsweise in mehreren Phasen erfolgen, um die Anforderungen der automatisierten Operationsdokumentation flexibel zu erfüllen. Zunächst wird vorzugsweise ein Basismodell des maschinellen Lernmodells bereitgestellt, das auf allgemeinen Daten trainiert wird, bevor es durch spezifische Operationsdaten feinabgestimmt wird. Dieser Prozess umfasst vorzugsweise die Phasen der Datensammlung, Datenvorbereitung, Modelltraining, Validierung und Optimierung, um eine hohe Genauigkeit und Robustheit zu gewährleisten.

Die Trainingsdaten für das maschinelle Lernmodell können vorzugsweise aus einer Vielzahl von Quellen stammen, wie Video- und Bildaufnahmen von realen Operationen, annotierten Operationsprotokollen oder synthetisch generierten Daten. Diese Daten könnten unterschiedliche Operationstätigkeiten umfassen, wie das Einsetzen eines Implantats, das Nähen einer Wunde oder das Entfernen eines Fremdkörpers, und könnten durch Metadaten wie Zeitstempel, Umgebungsmerkmale oder Sensorinformationen ergänzt werden. Die Trainingsdaten können vorzugsweise in Formaten wie MP4 oder AVI für Videodaten, JPEG oder PNG für Bilder und CSV oder JSON für zugehörige Annotationen vorliegen. Annotationen könnten dabei Labels enthalten, die den Daten semantische Bedeutungen wie "Implantat eingesetzt" oder "Wundverschluss durchgeführt" zuweisen.

Die Verarbeitung der Trainingsdaten durch das maschinelle Lernmodell erfolgt vorzugsweise durch eine Vorverarbeitung, die die Normalisierung der Bilddaten (z. B. Anpassung von Auflösung und Farbwerten), die Extraktion relevanter Merkmale (z. B. Bewegungsmuster oder Objektkonturen) und die Segmentierung der Szenen umfasst. Die annotierten Labels dienen vorzugsweise als Zielwerte für das Training des Modells. Während des Trainingsprozesses abstrahiert das Modell die Merkmale schrittweise, um die Beziehungen zwischen den Eingaben (Bilddaten) und den Ausgaben (Labels) zu erlernen.

Ein Foundation-Modellansatz könnte vorzugsweise zur Effizienzsteigerung genutzt werden. Dabei wird ein großes vortrainiertes Modell eingesetzt, das vorzugsweise auf umfangreichen, allgemeinen Datensätzen wie großen Video- oder Bilddatenbanken trainiert wurde. Dieses Foundation-Modell besitzt vorzugsweise ein breites Verständnis von allgemeinen visuellen und semantischen Mustern und reduziert die Notwendigkeit für umfangreiche, operationsspezifische Daten. Anschließend erfolgt vorzugsweise eine Feinabstimmung (Fine-Tuning) des Foundation-Modells mit domänenspezifischen Operationsdaten, wie annotierten Videos aus Operationssälen, um die spezifischen Anforderungen der Operationsdokumentation abzudecken.

Ein Anwendungsbeispiel des vorliegenden Verfahrens könnte vorzugsweise in einem Krankenhaus für die Dokumentation chirurgischer Eingriffe eingesetzt werden. In einem solchen Szenario könnte eine stationär installierte Kamera im Operationssaal vorzugsweise die Interaktionen zwischen dem Operateur und dem Patienten aufzeichnen. Das System erfasst vorzugsweise Szenen wie das Anreichen von Operationsutensilien, das Platzieren von Implantaten oder das Nähen einer Wunde. Die Kamera könnte vorzugsweise die Daten an das Bildverarbeitungsmodul übermitteln, das die Szenen in Echtzeit analysiert und relevante Bewegungsmuster erkennt. Ein maschinelles Lernmodell, das vorzugsweise speziell auf Operationsdaten trainiert wurde, könnte daraufhin Text-und/oder Sprachlabels wie "Implantat erfolgreich platziert" oder "Wundverschluss abgeschlossen" generieren.

Die generierten Labels könnten automatisch in das elektronische Operationsprotokoll des Krankenhauses integriert werden. Gleichzeitig könnte eine Dashboard-Schnittstelle dem OP-Team ermöglichen, die Dokumentation in Echtzeit zu überprüfen und gegebenenfalls zu korrigieren. Fehlerhafte Labels könnten vorzugsweise manuell angepasst werden, wobei diese Anpassungen in die Feedback-Schleife des maschinellen Lernmodells einfließen, um das System kontinuierlich zu optimieren. Dieses Beispiel zeigt, wie das Verfahren effizient eingesetzt werden kann, um den Dokumentationsaufwand zu reduzieren, die Genauigkeit zu erhöhen und das medizinische Personal zu entlasten.

Das maschinelle Lernmodell kann durch die Verwendung von zusätzlichen Audiodaten, die vorzugsweise mittels eines Mikrofons erfasst werden, weiter verbessert werden, da diese eine zusätzliche Dimension der Kontextinformationen liefern. Audiodaten könnten vorzugsweise Sprache, Umgebungsgeräusche und/oder spezifische akustische Ereignisse wie das Klirren chirurgischer Instrumente, das Öffnen von Verpackungen und/oder das Geräusch von medizinischen Geräten umfassen. Diese Informationen können vorzugsweise durch ein Mikrofon erfasst werden, das entweder in die Bildaufnahmeeinheit integriert ist, wie bei einer stationären Kamera im Operationssaal, oder als separates Gerät genutzt wird. Die Synchronisation von Audio- und Bilddaten könnte vorzugsweise zeitliche Übereinstimmungen zwischen visuellen und akustischen Signalen herstellen. So könnte das maschinelle Lernmodell durch das Hören eines Klammergeräuschs in Kombination mit einer visuellen Handlung erkennen, dass eine chirurgische Klammer angebracht wird.

Spracherkennung und/oder -analyse könnten vorzugsweise in das System integriert werden, um gesprochene Worte oder Sätze während einer Operation zu transkribieren. Dies kann vorzugsweise Hinweise auf die durchgeführte Handlung liefern, wie etwa, wenn der Operateur sagt: "Klammer ansetzen." Solche sprachlichen Hinweise könnten die visuelle Analyse ergänzen und das Modell bei der korrekten Interpretation der Handlung unterstützen. Darüber hinaus könnten Umgebungsgeräusche wie das Piepen eines Monitors, das Zischen eines Sauerstoffgeräts oder das Klicken eines Instruments analysiert werden. Diese Geräusche könnten vorzugsweise als Indikatoren für bestimmte operative Schritte dienen, etwa das Platzieren eines Endotrachealtubus oder das Öffnen einer Naht.

Die Kombination von Bild- und Audiodaten durch multimodale Datenfusion kann vorzugsweise eine ganzheitliche Analyse der Operationen ermöglichen. Transformer-Modelle oder andere spezialisierte Architekturen für multimodale Daten könnten vorzugsweise eingesetzt werden, um visuelle und akustische Informationen zusammenzuführen. Diese Fusion erlaubt es dem Modell vorzugsweise, unklare Informationen aus einem Datenstrom durch den anderen zu ergänzen. Trainingsdaten könnten vorzugsweise um annotierte Audioclips erweitert werden, die beispielsweise typische Geräusche und Sprachmuster während Operationen enthalten. Dadurch wird das Modell für akustische Variationen wie unterschiedliche Tonlagen und/oder Dialekte sensibilisiert.

Ein praktisches Beispiel zeigt die Vorteile dieser Integration. Angenommen, ein Operateur fixiert ein Implantat. Das visuelle Modell könnte vorzugsweise den Operateur und das Implantat erkennen, wäre jedoch möglicherweise nicht in der Lage, die spezifische Handlung eindeutig zu klassifizieren. Durch die Integration von Audiodaten könnte das Modell das Geräusch des Schraubens und die Aussage des Operateurs "Implantat fixiert" hören. Diese Informationen ermöglichen es dem Modell, das Label "Implantat fixiert" mit hoher Präzision zu generieren.

Durch diese Integration von Audiodaten könnte das maschinelle Lernmodell präziser und flexibler werden, insbesondere in Situationen, in denen die Sicht auf die Handlung eingeschränkt ist und/oder visuelle Daten allein nicht ausreichen. Die Kombination von visuellen und akustischen Signalen verbessert die Kontextualisierung und ermöglicht eine schnellere, zuverlässigere und umfassendere automatische Dokumentation von Operationen.

In einem weiteren Aspekt kann die Datensicherheit und Anonymität der Patienten vorzugsweise durch eine Reihe technischer Maßnahmen gewährleistet werden. Die erfassten Bilddaten von Interaktionen während der Operation können vorzugsweise vor der Verarbeitung durch den Bildverarbeitungsalgorithmus anonymisiert werden. Hierbei könnten Verfahren wie Gesichtserkennung und -maskierung zum Einsatz kommen, um Gesichter und/oder andere identifizierbare Merkmale automatisch zu erkennen und durch Unschärfe, Pixelation oder vollständige Abdeckung unkenntlich zu machen.

Um unbefugten Zugriff zu verhindern, könnten die Bilddaten während der Übertragung von der Bildaufnahmeeinheit an die Auswerte- und Recheneinheit durch Ende-zu-Ende-Verschlüsselung gesichert werden. Ein Verschlüsselungsverfahren wie AES-256 könnte vorzugsweise verwendet werden, um sicherzustellen, dass nur autorisierte Systeme die Daten entschlüsseln und weiterverarbeiten können. Ergänzend könnten pseudonymisierte Datenstrukturen genutzt werden, bei denen persönliche Identifikatoren, wie der Name des Patienten, durch eindeutige, nicht rückverfolgbare Codes ersetzt werden.

Die Speicherung der Daten könnte auf lokalen, geschützten Servern erfolgen, die physisch und digital gegen Angriffe abgesichert sind. Alternativ könnten Edge-Computing-Lösungen eingesetzt werden, bei denen die Verarbeitung und Anonymisierung der Daten bereits auf der Bildaufnahmeeinheit oder in einer lokalen Einheit stattfinden, sodass keine sensiblen Daten in externe Netzwerke übertragen werden müssen.

Zur Sicherstellung der Datensicherheit könnten Zugriffskontrollsysteme implementiert werden, die nur autorisierten Personen den Zugang zu bestimmten Datenbereichen erlauben. Diese Systeme könnten auf Zwei-Faktor-Authentifizierung (2FA) und/oder biometrischen Verfahren basieren. Zusätzlich könnten alle Datenoperationen durch ein Audit-Logging-System protokolliert werden, um verdächtige Zugriffsversuche zu identifizieren und gegebenenfalls zu blockieren.

Schließlich könnte ein datenschutzorientierter Ansatz durch den Einsatz von differenzieller Privatsphäre ergänzt werden, bei dem Rauschsignale zu den Daten hinzugefügt werden, um Rückschlüsse auf individuelle Patienten zu verhindern, während die nützliche Information für die Verarbeitung erhalten bleibt. Diese technischen Maßnahmen könnten eine sichere und anonymisierte Verarbeitung der Daten im Rahmen des beanspruchten Verfahrens gewährleisten.

### Kurze Beschreibung der Zeichnungen

Die beiliegenden Zeichnungen sollen ein weiteres Verständnis der Ausführungsformen der Erfindung vermitteln. Sie veranschaulichen Ausführungsformen und dienen im Zusammenhang mit der Beschreibung der Erklärung von Prinzipien und Konzepten der Erfindung.

Andere Ausführungsformen und viele der genannten Vorteile ergeben sich im Hinblick auf die Zeichnungen. Die dargestellten Elemente der Zeichnungen sind nicht notwendigerweise maßstabsgetreu zueinander gezeigt.
- Fig. 1: zeigt ein schematisches Flussdiagramm eines Ausführungsbeispiels des Verfahrens.
- Fig. 2: zeigt eine schematische Ansicht eines Blockdiagramms zu der vorliegenden Vorrichtung.
- Fig. 3: zeigt eine schematische Ansicht eines Blockdiagramms zu der vorliegenden Vorrichtung.

### Detaillierte Beschreibung der Zeichnungen

In den Figuren der Zeichnungen bezeichnen gleiche Bezugszeichen gleiche oder funktionsgleiche Elemente, Bauteile oder Komponenten, soweit nichts Gegenteiliges angegeben ist.

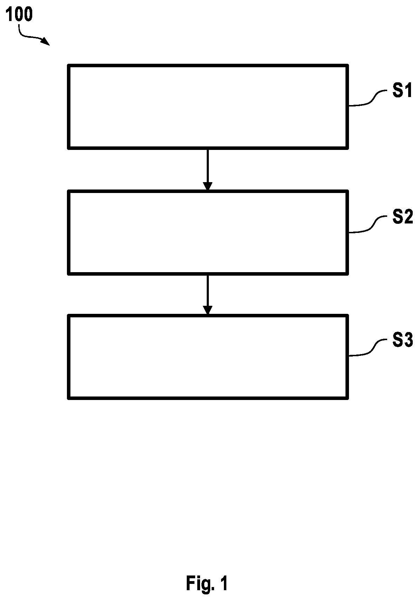

Fig. 1 zeigt ein schematisches Flussdiagramm eines Ausführungsbeispiels eines vorliegenden Verfahrens zur automatisierten Dokumentation von Operationen eines Patienten.

Das Verfahren kann in einer beliebigen Ausführungsform zumindest teilweise durch eine Vorrichtung 100 ausgeführt werden, die hierzu mehrere nicht näher dargestellte Komponenten, beispielsweise eine oder mehrere Bereitstellungseinrichtungen und/oder mindestens eine Auswerte- und Recheneinrichtung umfassen kann. Es versteht sich, dass die Bereitstellungseinrichtung gemeinsam mit der Auswerte- und Recheneinrichtung ausgebildet sein kann, oder von dieser unterschiedlich sein kann. Ferner kann die Vorrichtung 100, die ein Teil eines Systems sein kann, eine Speichereinrichtung und/oder eine Ausgabeeinrichtung und/oder eine Anzeigeeinrichtung und/oder eine Eingabeeinrichtung umfassen.

Das computerimplementierte Verfahren umfasst mindestens die folgenden Schritte:
In einem Schritt S1 erfolgt ein Erfassen von Bilddaten von Interaktionen zwischen einem Operateur und/oder einem Operationsutensil und einem Patienten unter Verwendung einer Bildaufnahmeeinheit.

In einem Schritt S2 erfolgt ein Verarbeiten der erfassten Bilddaten unter Verwendung eines Bildverarbeitungsalgorithmus zum Erkennen der Interaktionen in den Bilddaten.

In einem Schritt S3 erfolgt ein Generieren von Text- und/oder Sprachlabels zu den erkannten Interaktionen unter Verwendung eines maschinellen Labelgenerierung-Lernmodells zur automatisierten Dokumentation der Operation unter Verwendung der generierten Text- und/oder Sprachlabels.

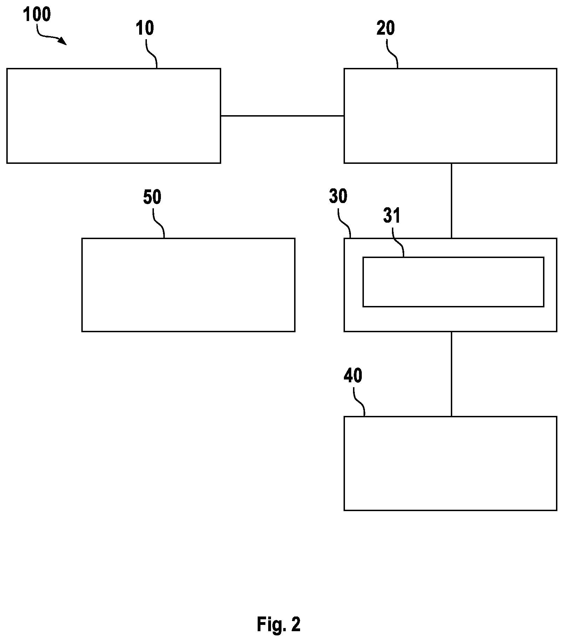

Fig. 2 zeigt eine schematische Darstellung der Architektur der Vorrichtung 100 zur automatisierten Dokumentation von Operationen. Die Vorrichtung 100 umfasst eine Bildaufnahmeeinheit 10, die visuelle Daten von Interaktionen 130 zwischen einem Operateur 110 und einem Patienten 120 erfasst. Die Bildaufnahmeeinheit 10 kann verschiedene Ausführungen umfassen, wie eine stationär installierte Kamera, eine tragbare Kamera oder eine in eine (Smart-) Brille integrierte Kamera. Die Bildaufnahmeeinheit 10 kann auch an einem Operationsutensil 110 oder am Kopf des Operateurs angeordnet sein. Die erfassten Daten werden vorzugsweise über eine Schnittstelle an eine Auswerte- und Recheneinheit 20 weitergeleitet.

Die Auswerte- und Recheneinheit 20 übernimmt die Analyse der Bilddaten und umfasst vorzugsweise verschiedene Module. Zunächst erfolgt die Verarbeitung der Daten durch ein Bildverarbeitungsmodul 25, das visuelle Merkmale extrahiert, wie beispielsweise die Position des Operateurs 110, des Patienten 120 und etwaiger Operationsutensilien. Die verarbeiteten Bilddaten werden anschließend im Labelgenerierungsmodul 30 analysiert, das mithilfe eines maschinellen Lernmodells 31 automatisch Text- und/oder Sprachlabels erzeugt. Diese Labels beschreiben die identifizierten Operationstätigkeiten, wie beispielsweise "Schnitt durchgeführt" oder "Naht angebracht".

Das Labelgenerierungsmodul 30 gibt die erzeugten Labels vorzugsweise an ein Ausgabemodul 40 weiter, das die Ausgabe vorzugsweise in unterschiedlichen Formaten bereitstellt. Das Ausgabemodul 40 ermöglicht vorzugsweise die Generierung von Textdokumenten, die direkt in elektronische Patientenakten integriert werden können, und/oder die Ausgabe von Sprachinformationen zur Unterstützung des Operateurs. Eine, insbesondere zentrale, Prozesseinheit 50 steuert vorzugsweise den gesamten Datenfluss zwischen den einzelnen Modulen und stellt sicher, dass die Daten konsistent verarbeitet und weitergeleitet werden. Die Architektur ermöglicht eine automatisierte, präzise und effiziente Dokumentation von Operationen.

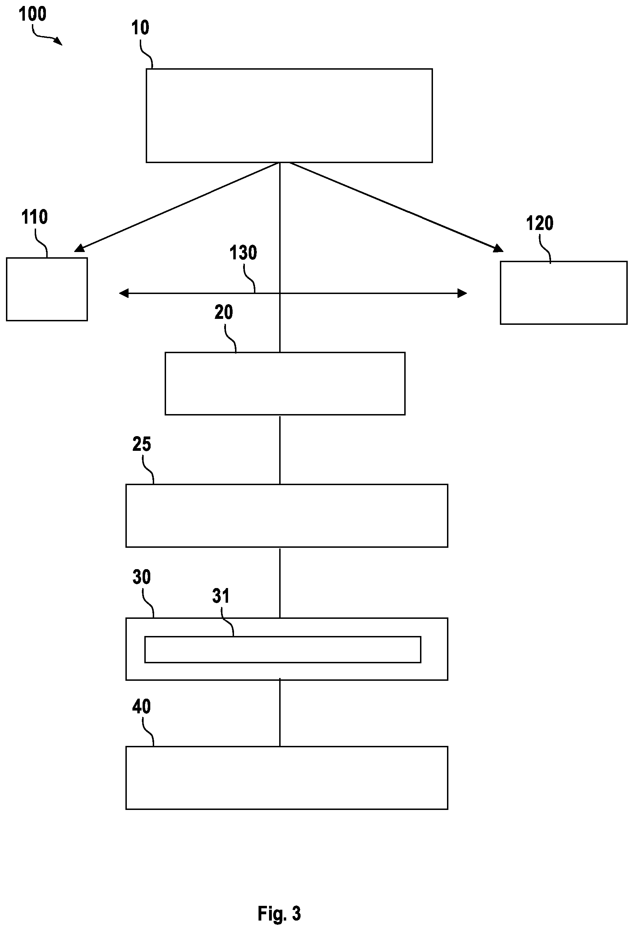

Fig. 3 zeigt die räumliche Anordnung der einzelnen Systemkomponenten in einem typischen Operationsumfeld. In der Mitte der Darstellung ist ein Patient 120 schematisch zu sehen, der von einem Operateur 110 operiert wird. Die Interaktionen 130 zwischen dem Operateur 110 und dem Patienten 120 werden durch eine Bildaufnahmeeinheit 10 erfasst. Diese Bildaufnahmeeinheit 10 kann als tragbare Komponente, beispielsweise in Form einer Smart-Brille, oder als stationär installierte Kamera im Operationssaal ausgeführt sein.

Die Bildaufnahmeeinheit 10 zeichnet die visuellen Daten der Interaktionen 130 auf und synchronisiert diese vorzugsweise mit zusätzlichen Informationen, wie Bewegungs- oder Umgebungsdaten. Die erfassten Daten werden an die Auswerte- und Recheneinheit 20 übermittelt, die beispielsweise Bewegungen, Objekte und/oder Handlungen analysiert. Beispielsweise kann erkannt werden, ob der Operateur 110 einen Schnitt ausführt, ein Implantat platziert und/oder medizinische Geräte verwendet.

Nach der Analyse der Interaktionen 130 in der Auswerte- und Recheneinheit 20 werden die Ergebnisse vorzugsweise an das Labelgenerierungsmodul 30 weitergeleitet. Das Labelgenerierungsmodul 30 nutzt mindestens ein maschinelles Lernmodell oder eine Modellkomposition aus mehreren maschinellen und/oder statistischen und/oder analytischen Modellen, um die Daten in, insbesondere aussagekräftige, Labels zur Dokumentation der Operation zu übersetzen. Diese Labels könnten beispielsweise "Implantat platziert", "Schnitt geschlossen" oder "Instrument entfernt" lauten. Die Labels werden vorzugsweise automatisch im Kontext der Operation erstellt und an das Ausgabemodul 40 übermittelt. Das Ausgabemodul 40 gibt dann vorzugsweise die Dokumentation der Operation unter Verwendung der generierten Labels in strukturierter Form aus. Dabei können vorzugsweise auch allgemeine Kontextinformationen und/oder Vorlage-Informationen zu dem Aufbau und/oder der Art der Dokumentation auf Basis von Sprachverarbeitung und/oder unter Verwendung eines Sprachmodells erzeugt werden. Diese Ausgabe erfolgt entweder als Textdokument, das beispielsweise in einer digitalen Patientenakte gespeichert wird, und/oder als Sprachdatei, die den Operateur auditiv unterstützt.

### Bezugszeichenliste

- 10: Bildaufnahmeeinheit
- 20: Auswerte- und Recheneinheit
- 25: Bildverarbeitungsmodul
- 30: Labelgenerierungsmodul
- 31: Maschinelles Lernmodell
- 40: Ausgabemodul
- 50: Prozesseinheit
- 100: Vorrichtung
- 110: Operateur und/oder Operationsutensil
- 120: Patient
- 130: Interaktionen
- S1: Schritt 1 (Erfassen von Bilddaten)
- S2: Schritt 2 (Verarbeiten von Bilddaten)
- S3: Schritt 3 (Generieren von Labels)

## Patentansprüche

1. Verfahren zur automatisierten Dokumentation einer Operation eines Patienten (120), aufweisend die Schritte:
- Erfassen (S1) von Bilddaten von Interaktionen (130) zwischen einem Operateur und/oder einem Operationsutensil (110) und einem Patienten (120) unter Verwendung einer Bildaufnahmeeinheit (10);
- Verarbeiten (S2) der erfassten Bilddaten unter Verwendung eines Bildverarbeitungsalgorithmus zum Erkennen der Interaktionen in den Bilddaten;
- Generieren (S3) von Text- und/oder Sprachlabels zu den erkannten Interaktionen (130) unter Verwendung eines maschinellen Lernmodells (31) zur automatisierten Dokumentation der Operation unter Verwendung der generierten Text- und/oder Sprachlabels, und **gekennzeichnet durch**
- Erzeugen einer Warnmeldung unter Verwendung der generierten Text- und/oder Sprachlabels, wenn eine Gefahr besteht, dass ein Operationsutensil (110) in einem Körper des Patienten (120) verbleiben könnte.

2. Verfahren nach Anspruch 1, wobei die Bildaufnahmeeinheit (10) eine Kamera zur Bild- und/oder Videoerfassung aufweist, und wobei die Bildaufnahmeeinheit (10) in einem Raum, in dem der Patient (120) operiert wird, insbesondere in einer Operationsleuchte und/oder an einem Körper des Operateurs und/oder an dem Operationsutensil (110), angeordnet ist.

3. Verfahren nach Anspruch 1 oder 2, wobei der Bildverarbeitungsalgorithmus eine Segmentierung der Bilddaten und/oder eine Objekterkennung und/oder eine Bewegungsanalyse und/oder eine Aktivitätserkennung und/oder eine Gesichtserkennung und/oder eine Anomalieerkennung und/oder eine Objektverfolgung und/oder eine Klassifikation von Handlungen und/oder eine multimodale Bildverarbeitung und/oder eine Feature-Extraktion aufweist.

4. Verfahren nach einem der Ansprüche 1 bis 3, wobei der Bildverarbeitungsalgorithmus ein maschinelles Lernmodell zur Bildverarbeitung aufweist.

5. Verfahren nach einem der Ansprüche 1 bis 4, wobei das maschinelle Lernmodell unter Verwendung von, insbesondere international standardisierten, Operationsprotokollen zu Operationen an Patienten (120) und damit verbundenen Bilddaten trainiert wird oder zumindest feinabgestimmt wird.

6. Verfahren nach einem der Ansprüche 1 bis 5, wobei eine Ausgabe der generierten Text- und/oder Sprachlabels als Audio- und/oder Textdatei erfolgt.

7. Verfahren nach einem der Ansprüche 1 bis 6, wobei fehlerhafte Labels in einer Revision der OP-Dokumentation identifiziert und nachfolgend dazu verwendet werden, das maschinelle Lernmodell und/oder den Bildverarbeitungsalgorithmus nachzutrainieren.

8. Computerprogrammprodukt, umfassend Befehle, die bei der Ausführung des Programms durch einen Computer diesen veranlassen, die Schritte des Verfahrens nach einem der Ansprüche 1 bis 7 auszuführen.

9. Vorrichtung (100) zur automatisierten Dokumentation einer Operation eines Patienten, wobei die Vorrichtung (100) eine Auswerte- und Recheneinrichtung (20) aufweist, die dazu ausgebildet ist, die folgenden Schritte auszuführen:
- Erfassen (S1) von Bilddaten von Interaktionen (130) zwischen einem Operateur und/oder einem Operationsutensil (110) und einem Patienten (120) unter Verwendung einer Bildaufnahmeeinheit (10);
- Verarbeiten (S2) der erfassten Bilddaten unter Verwendung eines Bildverarbeitungsalgorithmus zum Erkennen der Interaktionen (130) in den Bilddaten;
- Generieren (S3) von Text- und/oder Sprachlabels zu den erkannten Interaktionen (130) unter Verwendung eines maschinellen Lernmodells (31) zur automatisierten Dokumentation der Operation unter Verwendung der generierten Text- und/oder Sprachlabels, und **gekennzeichnet durch**
- Erzeugen einer Warnmeldung unter Verwendung der generierten Text- und/oder Sprachlabels, wenn eine Gefahr besteht, dass ein Operationsutensil (110) in einem Körper des Patienten (120) verbleiben könnte.

## Claims

1. A method for the automated documentation of an operation of a patient (120), comprising the steps of:
capturing (S1) image data of interactions (130) between an operator and/or a surgical item (110) and a patient (120) using an image capture unit (10);
processing (S2) the captured image data using an image processing algorithm for detecting the interactions in the image data;
generating (S3) text and/or speech labels for the detected interactions (130) using a machine-learning model (31) for the automated documentation of the operation using the generated text and/or speech labels, and **characterized by**
generating a warning message using the generated text and/or speech labels when there is a risk that a surgical item (110) could remain in a body of the patient (120).

2. The method according to claim 1, wherein the image capture unit (10) comprises a camera for image and/or video capture, and wherein the image capture unit (10) is arranged in a room in which the patient (120) is operated on, in particular in an operating light and/or on a body of the operator and/or on the surgical item (110).

3. The method according to claim 1 or 2, wherein the image processing algorithm comprises image-data segmentation and/or object recognition and/or movement analysis and/or activity recognition and/or facial recognition and/or anomaly detection and/or object tracking and/or classification of actions and/or multimodal image processing and/or feature extraction.

4. The method according to any one of claims 1 to 3, wherein the image processing algorithm comprises a machine-learning model for image processing.

5. The method according to any one of claims 1 to 4, wherein the machine-learning model is trained or at least fine-tuned using operation protocols for operations on patients (120), in particular internationally standardized operation protocols, and associated image data.

6. The method according to any one of claims 1 to 5, wherein an output of the generated text and/or speech labels is provided as an audio and/or text file.

7. The method according to any one of claims 1 to 6, wherein erroneous labels are identified in a review of the operation documentation and subsequently used to retrain the machine-learning model and/or the image processing algorithm.

8. A computer program product, comprising instructions which, when the program is executed by a computer, cause the computer to carry out the steps of the method according to any one of claims 1 to 7.

9. A device (100) for the automated documentation of an operation of a patient, wherein the device (100) comprises an evaluation and computing device (20) configured to carry out the following steps:
capturing (S1) image data of interactions (130) between an operator and/or a surgical item (110) and a patient (120) using an image capture unit (10);
processing (S2) the captured image data using an image processing algorithm for detecting the interactions (130) in the image data;
generating (S3) text and/or speech labels for the detected interactions (130) using a machine-learning model (31) for the automated documentation of the operation using the generated text and/or speech labels, and **characterized by**
generating a warning message using the generated text and/or speech labels when there is a risk that a surgical item (110) could remain in a body of the patient (120).

## Revendications

1. Procédé de documentation automatisée d'une opération d'un patient (120), comprenant les étapes consistant à :
acquérir (S1) des données d'image d'interactions (130) entre un opérateur et/ou un article chirurgical (110) et un patient (120) au moyen d'une unité de capture d'images (10) ;
traiter (S2) les données d'image acquises au moyen d'un algorithme de traitement d'images destiné à détecter les interactions dans les données d'image ;
générer (S3) des étiquettes textuelles et/ou vocales relatives aux interactions détectées (130) au moyen d'un modèle d'apprentissage automatique (31) destiné à la documentation automatisée de l'opération au moyen des étiquettes textuelles et/ou vocales générées, et **caractérisé par**
la génération d'un message d'avertissement au moyen des étiquettes textuelles et/ou vocales générées lorsqu'il existe un risque qu'un article chirurgical (110) puisse rester dans un corps du patient (120).

2. Procédé selon la revendication 1, dans lequel l'unité de capture d'images (10) comprend une caméra destinée à la capture d'images et/ou de vidéos, et dans lequel l'unité de capture d'images (10) est disposée dans une pièce dans laquelle le patient (120) est opéré, en particulier dans une lampe opératoire et/ou sur le corps de l'opérateur et/ou sur l'article chirurgical (110).

3. Procédé selon la revendication 1 ou 2, dans lequel l'algorithme de traitement d'images comprend une segmentation des données d'image et/ou une reconnaissance d'objets et/ou une analyse de mouvements et/ou une reconnaissance d'activités et/ou une reconnaissance faciale et/ou une détection d'anomalies et/ou un suivi d'objets et/ou une classification d'actions et/ou un traitement d'images multimodal et/ou une extraction de caractéristiques.

4. Procédé selon l'une quelconque des revendications 1 à 3, dans lequel l'algorithme de traitement d'images comprend un modèle d'apprentissage automatique destiné au traitement d'images.

5. Procédé selon l'une quelconque des revendications 1 à 4, dans lequel le modèle d'apprentissage automatique est entraîné ou au moins ajusté finement au moyen de protocoles opératoires relatifs à des opérations pratiquées sur des patients (120), en particulier de protocoles opératoires standardisés au niveau international, et de données d'image associées.

6. Procédé selon l'une quelconque des revendications 1 à 5, dans lequel une sortie des étiquettes textuelles et/ou vocales générées est fournie sous la forme d'un fichier audio et/ou texte.

7. Procédé selon l'une quelconque des revendications 1 à 6, dans lequel des étiquettes erronées sont identifiées lors d'une révision de la documentation de l'opération et utilisées ensuite pour réentraîner le modèle d'apprentissage automatique et/ou l'algorithme de traitement d'images.

8. Produit-programme informatique, comprenant des instructions qui, lors de l'exécution du programme par un ordinateur, amènent l'ordinateur à exécuter les étapes du procédé selon l'une quelconque des revendications 1 à 7.

9. Dispositif (100) destiné à la documentation automatisée d'une opération d'un patient, dans lequel le dispositif (100) comprend un dispositif d'évaluation et de calcul (20) configuré pour exécuter les étapes suivantes:
acquérir (S1) des données d'image d'interactions (130) entre un opérateur et/ou un article chirurgical (110) et un patient (120) au moyen d'une unité de capture d'images (10);
traiter (S2) les données d'image acquises au moyen d'un algorithme de traitement d'images destiné à détecter les interactions (130) dans les données d'image;
générer (S3) des étiquettes textuelles et/ou vocales relatives aux interactions détectées (130) au moyen d'un modèle d'apprentissage automatique (31) destiné à la documentation automatisée de l'opération au moyen des étiquettes textuelles et/ou vocales générées, et **caractérisé par**
la génération d'un message d'avertissement au moyen des étiquettes textuelles et/ou vocales générées lorsqu'il existe un risque qu'un article chirurgical (110) puisse rester dans un corps du patient (120).
